# EUROPÄISCHE PATENTSCHRIFT

(11) **EP 1 231 194 B1**
(45) Veröffentlichungstag und Bekanntmachung des Hinweises auf die Patenterteilung: **12.11.2003**
(21) Anmeldenummer: 02000931.2
(22) Anmeldetag: 16.01.2002
(51) Int. Cl.: C07C 11/02

(54) **Herstellung von 1-Olefinen**
Preparation of 1-olefins
Préparation des 1-oléfines

(30) Priorität: 10.02.2001 DE 10106185; 27.09.2001 DE 10147775
(43) Veröffentlichungstag der Anmeldung: 14.08.2002
(73) Patentinhaber: Oxeno Olefinchemie GmbH, 45764 Marl (DE)
(72) Erfinder: Wiese, Klaus-Diether, Dr., 45721 Haltern (DE); Protzmann, Guido, Dr., 64673 Zwingenberg (DE); Kaizik, Alfred, Dr., 45772 Marl (DE); Büschken, Wilfried, Dr., 45721 Haltern (DE)

(56) Entgegenhaltungen:
- GB-A- 1 233 020

## Beschreibung

Die Erfindung betrifft die Herstellung von 1-Olefinen aus Aldehyden durch eine dreistufige Synthese.

Olefine zählen wegen ihrer Reaktivität zu den wichtigsten Synthesebausteinen der organischen Chemie. Sie sind Vorstufen für eine Vielzahl von Verbindungen, wie beispielsweise Aldehyde, Ketone, Alkohole, Carbonsäuren und Halogenverbindungen. In großen Mengen werden sie zur Herstellung von Homo- oder Cooligomeren und Homo- und Copolymeren verwendet, wie beispielweise Polyethylen oder Polypropylen.

Ethylen und Propylen werden durch Steamcracking oder durch katalytische Spaltung von Kohlenwasserstoffen weltweit in großen Mengen hergestellt. Dabei fallen beträchtliche Mengen an C₄-Olefinen ( Isobuten, 1-Buten , 2-Butene) und in geringerem Umfang C₅-Olefine an. Höhere 1-Olefine werden meistens durch Aufbaureaktionen erzeugt.

Ethylen kann mit Hilfe von Ziegler-Katalysatoren oligomerisiert werden, wobei ein Gemisch von unverzweigten 1-Olefinen mit gerader C-Zahl anfällt.

Nach einer Variante des SHOP-Prozesses können aus Ethylen unverzweigte 1-Olefine mit gerader und ungerader C-Zahl hergestellt werden. Dieses Verfahren umfasst drei Reaktionsschritte, nämlich Ethylenoligomerisierung, Isomerisierung, d. h. Verschiebung der Doppelbindungen, und Kreuzmethathese des Olefingemisches mit innenständigen Doppelbindungen mit Ethylen.

Durch Dehydrierung von geradkettigen Paraffinen, beispielsweise durch Chlorierung und Dehydrochlorierung, entstehen Olefine mit überwiegend innenständigen Doppelbindungen, die durch Kreuzmetathese zu 1-Olefinen umgesetzt werden können.

Die obengenannten Verfahren haben den Nachteil gemeinsam, dass immer eine Vielzahl an 1-Olefinen erzeugt wird.

Geradkettige 1-Olefine mit gerader C-Zahl können aus Fettalkoholen durch Wasserabspaltung gewonnen werden. Nachteilig daran ist der hohe Preis der Einsatzstoffe und dass im Wesentlichen nur Fettalkohole mit 12 bis 18 C-Atomen in ausreichendem Maße zur Verfügung stehen.

Da die bekannten Verfahren nicht alle gewünschten 1-Olefine in ausreichend großer Menge und/oder in genügend hoher Reinheit liefern, besteht ein Bedarf, auf Basis von leicht zugänglichen Ausgangsstoffen 1-Olefine herzustellen.

Es wurde nun gefunden, dass 1-Olefine aus Aldehyden durch Aldolkondensation mit Aceton, durch Hydrierung der α,β-ungesättigten Ketone zu den gesättigten Alkoholen und durch anschließende Wasserabspaltung aus den Alkoholen hergestellt werden können.

Die Erfindung ist demnach ein Verfahren zur Herstellung von 1-Olefinen mit 7 bis 24 C-Atomen aus Aldehyden mit 4 bis 21 C-Atomen, dadurch gekennzeichnet, dass ein Aldehyd mit Aceton zum α,β-ungesättigten Keton kondensiert, dass das so erhaltene ungesättigte Keton zum gesättigten Alkohol hydriert und dass aus dem gesättigten Alkohol Wasser abgespaltet wird.

Im erfindungsgemäßen Verfahren können Aldehyde oder Aldehydgemische mit 4 bis 21 C-Atomen eingesetzt werden. Die eingesetzten Aldehyde können aus verschiedenen Quellen stammen. Es können Aldehyde eingesetzt werden, die durch Dehydratisierung von Alkoholen, beispielsweise Fettalkoholen, gewonnen worden sind. Ebenfalls können Aldehyde aus Spaltungsreaktionen, beispielsweise Heptanal aus Ricinolsäuremethylester, als Ausgangsstoff verwendet werden. Insbesondere können Aldehyde, die durch Hydroformylierung von Olefinen erzeugt worden sind, eingesetzt werden.

Darüber hinaus können auch ungesättigte Aldehyde, die durch Selbstkondensation eines Aldehydes, wie 2-Ethylhex-2-enal aus n-Butyraldehyd, entstanden sind, verwendet werden.

Beispielsweise können die im Folgenden genannten Aldehyde Ausgangsstoff fiir das erfindungsgemäße Verfahren sein:
n-Butyraldehyd, Isobutyraldehyd, Crotonaldehyd, Valeraldehyd, 2-Methylbutanal, 3-Methylbutanal, Dimethylpropanal, Tiglinaldehyd, 3,3-Dimethylacrolein, n-Hexanal, Isohexanal, n-Heptanal, Citral, α- und β-Citral, Benzaldehyd, Zimtaldehyd, Phenylacetaldehyd, Hydrozimtaldehyd, 2-Phenylpropionaldehyd, Cyclohexylcarbaldehyd, Anisaldehyd.

Aldehydgemische, hergestellt durch Hydroformylierung von Dipropen, Dibuten, Tripropen, Tetrapropen, Tributen, Pentapropen, Tetrabuten.

Ein bevorzugter Einsatzstoff ist n-Pentanal.

Die Aldolkondensation von Aldehyden mit Aceton zu α,β-ungesättigten Ketone wird vorzugsweise als Zweiphasenreaktion durchgeführt. Die Umsetzung erfolgt, wie in DE 199 57 522 beschrieben, auf deren Offenbarung hier ausdrücklich hingewiesen wird, in einem Rohrreaktor, wobei der Katalysator in der kontinuierlichen Phase und das Edukt in einer dispersen Phase enthalten ist und der Belastungsfaktor B des Reaktors gleich oder größer 0,8 und das Massenverhältnis zwischen Katalysatorphase und organischer Phase größer 2 ist. (Der Belastungsfaktor B ist wie folgt definiert: B = PD/PS. PD [Pa/m] ist ein längenbezogener Druckverlust über den Reaktor unter Betriebsbedingungen und PS [Pa/m] eine Rechengröße mit der Dimension eines längenbezogenen Druckes, definiert als Verhältnis von Massenstrom M [kg/s] aller Komponenten im Reaktor zum Volumenstrom V [m³/s] aller komponenten unter Betriebsbedingungen, multipliziert mit g =9,81 [m/s²], d. h. PS =(M/V)*g .) Als Katalysatorphasen werden bevorzugt wässrige Lösungen von Hydroxiden, Hydrogencarbonaten, Carbonaten oder Carboxylaten in Form ihrer Alkali- oder Erdalkaliverbindungen verwendet, insbesondere Natron- und Kalilauge. Die Konzentration des Katalysators in der Katalysatorlösung liegt zwischen 0,1 und 15 Massen-% , insbesondere zwischen 0,1 und 5 Massen-%.Aldehyd, Aceton und optional ein Lösungsmittel werden vor dem Reaktor in die Katalysatorphase eingespeist. Das molare Verhältnis zwischen Aldehyd und Aceton beträgt 5/1 bis 1/10, vorzugsweise 1/1 bis 1/5 . Die Umsetzung erfolgt in einem Temperaturbereich 40 °C bis 150 °C, vorzugsweise im Bereich 50 °C bis 120 °C. Die Reaktionszeit liegt zwischen 0,1 und 20 Minuten, vorzugsweise zwischen 0,2 und 10 Minuten. Vom Reaktionsaustrag wird die Katalysatorphase abgetrennt und in den Reaktor zurückgeführt. Vorzugsweise werden vor der Phasentrennung nicht umgesetzte Einsatzstoffe, etwas Produkt, Wasser und optional Lösungsmittel abdestilliert. Das Destillat trennt sich nach Kondensation in eine wässrige und eine organische Phase, die in den Reaktor zurückgeführt wird. Die wässrige Phase wird nach destillativer Abtrennung von Edukten, insbesondere Aceton, zum Teil zur Ausschleusung des Reaktionswassers verworfen und zum Teil nach optionaler Verwendung als Waschflüssigkeit in den Prozess zurückgeführt. Die vom Katalysator abgetrennte Produktphase kann gegebenenfalls nach einer Wasserwäsche destillativ zu reinem α,β-ungesättigten Keton aufgearbeitet werden. Eine andere Möglichkeit besteht darin, das Rohprodukt in der nächsten Stufe einzusetzen. Durch diese Verfahrensweise gelingt es, das gewünschte α,β-ungesättigte Keton in hoher Selektivität herzustellen.

Ein gegebenenfalls dem Edukt oder dem Reaktionsaustrag zugesetztes organisches Lösungsmittel muss folgende Eigenschaften besitzen: Es löst Produkte und Edukte und ist selbst in der Katalysatorphase kaum löslich. Es verhält sich in der Aldolkondensation und optional in der Hydrierung inert. Es kann von dem Zwischenprodukt, dem α,β-ungesättigten Keton, und/oder dem Folgeprodukt, dem gesättigten Alkohol, destillativ abgetrennt werden. Bevorzugte Lösungsmittel sind diejenigen, die mit Wasser ein Minimum- Hetero-Azeotrop bilden, sodass Wasser leicht vom α,β-ungesättigten Keton durch Destillation abgetrennt werden kann. Geeignete Lösungsmittel sind beispielsweise Ether, Kohlenwasserstoffe, wie Cyclohexan oder Toluol.

Das als Zwischenprodukt anfallende ungesättigte Keton kann nicht nur, wie im erfindungsgemäßen Verfahren beansprucht, zu 1-Olefin umgesetzt werden, sondern auch für andere Synthesen genutzt werden. So könnte daraus durch Hydrierung ein gesättigtes Keton hergestellt werden, das mit einem Aldehyd zu einem neuen ungesättigten Keton umgesetzt werden könnte. Dieses Produkt könnte Zwischenprodukt für einen gesättigten Alkohol oder einem Olefin mit innenständiger Doppelbindung sein.

Das nach dem erfindungsgemäßen Verfahren durch gekreuzte Aldolkondensation gewonnene α,β-ungesättigte Keton wird in reiner Form oder als Gemisch, das Aceton, Ausgangsaldehyd, Wasser, Lösungsmittel und Hochsieder enthalten kann, zu den entsprechenden gesättigten Alkoholen hydriert.

Die Hydrierung wird bevorzugt in der Flüssigphase durchgeführt.

Zur Hydrierung können Katalysatoren oder Katalysatorsysteme verwendet werden, die sowohl olefinische Doppelbindungen als auch Carbonylgruppen hydrieren. Für die Hydrierung der α,β-ungesättigte Ketone sind insbesondere diejenigen Katalysatoren geeignet, die in der Technik für die Hydrierung von 2-Ethylhex-2-enal zu 2-Ethylhexanol eingesetzt werden.

Man kann zur Hydrierung z. B. Kupfer/Nickel-, Kupfer/Chrom-, Kupfer/Chrom/Nickel-, Zink/Chrom-, Nickel/Molybdän-Katalysatoren verwenden. Es können auch Kombinationen von zwei oder mehreren Katalysatoren verwendet werden. Die Katalysatoren können trägerfrei sein, oder die hydrieraktiven Stoffe bzw. ihre Vorläufer können auf Träger, wie beispielsweise Siliciumdioxid oder Aluminiumdioxid, aufgebracht sein. Das α,β-ungesättigte Keton kann insbesondere an einem im Festbett angeordneten Katalysator, der die Metalle Kupfer, Chrom und Nickel enthält, zum entsprechenden gesättigten Alkohol hydriert werden.

Bevorzugte Katalysatoren, an denen die α,β-ungesättigten Ketone hydriert werden, enthalten, jeweils 0,3- 15 Massen-% Kupfer und Nickel sowie als Aktivatoren 0,05 - 3,5 Massen-% Chrom und vorteilhaft 0,01 - 1,6 Massen-%, vorzugsweise 0,02 - 1,2 Massen-% einer Alkalikomponente auf einem Trägermaterial, vorzugsweise Aluminiumoxid und Siliciumdioxid. Die Mengenangaben beziehen sich auf den noch nicht reduzierten Katalysator. Die Alkalikomponente ist optional.

Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudate oder Ringen. Sie werden zweckmäßig vor ihrem Einsatz aktiviert, z. B. durch Erhitzen im Wasserstoffstrom.

Die Hydrierung, bevorzugt eine Flüssigphasenhydrierung, wird im Allgemeinen unter einem Gesamtdruck von 5 bis 200 bar, insbesondere von 5 bis 30 bar ganz besonders 15 bis 25 bar durchgeführt. Eine Hydrierung in der Gasphase kann auch bei niedrigeren Drücken durchgeführt werden, mit entsprechend großen Gasvolumina. Werden mehrere Hydrierungsreaktoren eingesetzt, können die Gesamtdrücke in den einzelnen Reaktoren innerhalb der genannten Druckgrenzen gleich oder verschieden sein.

Die Reaktionstemperaturen liegen bei Hydrierung in flüssiger oder gasförmiger Phase in der Regel zwischen 120 und 220 °C, insbesondere zwischen 140 und 180 °C. Beispiele für solche Hydrierungen sind in den Patentanmeldungen EP 0 470 344 A2 und EP 0 326 674 A2 beschrieben.

Optional kann die Hydrierung von 3-Octen-2-on zu Octan-2-ol zweistufig durchgeführt werden. Dabei wird in der ersten Stufe beispielsweise an einem Palladiumkatalysator die olefinische Doppelbindung und in der zweiten Stufe an einem der oben genannten Kontakte die Carbonylgruppe hydriert.

Die in der zweiten Reaktionsstufe gewonnen gesättigten Alkohole werden in die dritte Stufe des erfindungsgemäßen Verfahrens eingesetzt. Darüber hinaus können sie auch als Lösemittel oder zur Herstellung von Weichmacher oder Detergenzien verwendet werden.

Die Wasserabspaltung aus 2-Alkoholen liefert im Allgemeinen ein Gemisch aus 1- und 2-Olefinen. Die katalytische Dehydratisierung von 2-Alkoholen zu überwiegend 1-Olefinen ist aus der Literatur bekannt. So werden beispielsweise Gasphasenverfahren, bei denen die Wasserabspaltung im Temperaturbereich von 250 bis 350 °C an Aluminiumoxid oder Zirkoniumoxiden erfolgt, in US 5 130 287, US 5 210 363, GB 1 225 559 und 1 233 020 offengelegt.

Dementsprechend wird im erfindungsgemäßen Verfahren die Herstellung der 1-Olefine durch Dehydratisierung von 2-Alkoholen an im Festbett angeordneten Katalysatoren in der Gas- oder Gas-Flüssig-Mischphase durchgeführt.

Das Reaktionsgemisch kann, gegebenenfalls nach Wasserabtrennung, destillativ in Ausgangsalkohol, Olefine und Nebenprodukte getrennt werden. Der nicht umgesetzte Alkohol kann in die Dehydratisierungsstufe zurückgeführt werden.

Aus der Olefinfraktion kann, wenn sie auch andere Olefine als das Zielprodukt enthält, durch Destillation das reine 1-Olefin gewonnen werden.

Die nach dem erfindungsgemäßen Verfahren gewonnenen 1-Olefine können als Monomere oder Comonomere zur Herstellung von Oligomeren oder Polymeren eingesetzt werden. Sie sind Ausgangsverbindungen für die Herstellung von Epoxiden, Ketonen, Aldehyden, Alkoholen und Carbonsäuren. Weiterhin können sie als Alkylierungsmittel oder als Komponente in Enreaktionen verwendet werden. Monoverzweigte Olefine, insbesondere die am zweiten oder vorletzten C-Atom Methyl-verzweigten, eignen sich zur Herstellung von tertiären Carbonsäuren nach der Kochsynthese. Dabei kommt es nicht auf die Lage der Doppelbindung an, sondern auf die Art der Verzweigung, die durch das erfindungsgemäße Verfahren mitbestimmt wird.

Der Vorteil des erfindungsgemäßen Verfahrens besteht darin, dass 1-Olefine aus leicht zugänglichen Aldehyden hergestellt werden können, wobei der Verzweigungsgrad des hergestellten Olefins dem des Ausgangsaldehyds entspricht. So kann beispielsweise 1-Octen aus n-Pentanal, dass durch Hydroformylierung von linearen Butenen gewonnen werden kann, hergestellt werden.

Die folgenden Beispiele sollen die Erfindung erläutern, ohne ihre Anwendungsbreite einzuschränken, die sich aus der Beschreibung und den Patentansprüchen ergibt.

### Beispiel 1 - Kondensation von Aceton und Pentanal zu 3-Octen-2-on

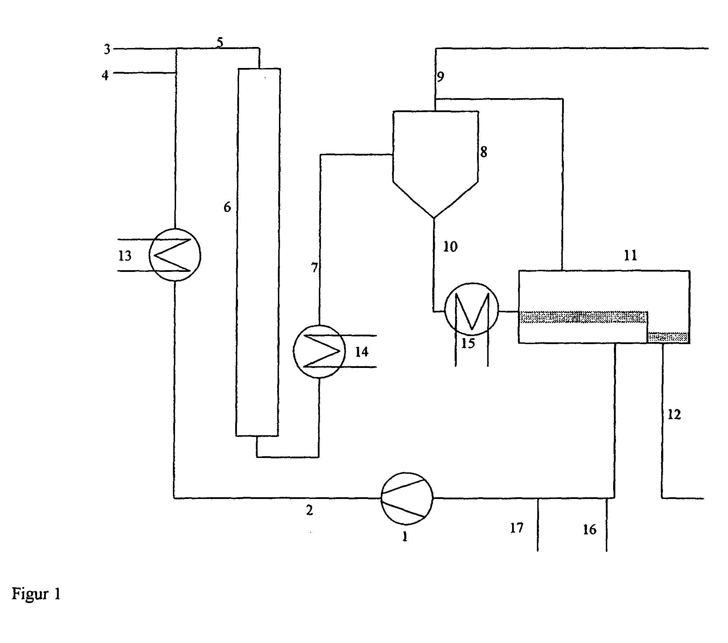

Die dem Beispiel 1 beigefügte erste Tabelle beschreibt zunächst die Katalysatorzusammensetzung, dann die Menge des Eduktes und dessen Zusammensetzung.
Im unteren Bereich der zweiten Tabelle ist die Produktzusammensetzung aufgelistet.
Im oberen Bereich der zweiten Tabelle sind die Raum-Zeit-Ausbeute (RZA), der Umsatz (U) der Aldehyde, die Selektivität (S) zu den gewünschten Aldolkondensationsprodukten und der Belastungsfaktor (B) angegeben. Bei der beschriebenen Katalysatorzusammensetzung ist zu beachten, dass es sich bei den Beispielen um Startwerte handelt. Der Anteil an NaOH wurde durch das Reaktionswasser der Aldolkondensation leicht verdünnt. Darüber hinaus führt die parallel zur Aldolkondensation ablaufende Cannizzaro-Reaktion zur Neutralisation des alkalischen Katalysators. Beide Effekte sind im beobachteten Zeitraum aber so gering, dass dies für die Beschreibung des Versuchs und des Versuchsergebnisses unwesentlich ist.
Die Aldolisierung erfolgte in einer Versuchsapparatur, die schematisch in Fig. 1 dargestellt ist. Hierin wird mit einer Pumpe 1 die kontinuierliche Katalysatorphase 2 im Kreislauf gepumpt. Zum Katalysator wird der Aldehyd oder die Aldehydmischung durch Leitung 3 oder verschiedene Aldehyde getrennt durch die Leitungen 3 und 4 zugemischt. Für das nachfolgend aufgeführte Beispiel wurden die Edukte ausschließlich über Leitung 3 zugemischt. Die Mehrphasenmischung 5 wird durch den Rohrreaktor 6 mit einer Länge von 3 m und einem Durchmesser von 17,3 mm gepumpt, der mit statischen Mischelementen mit einem hydraulischen Durchmesser von 2 mm versehen war. Die resultierende Mischung 7, bestehend aus dem Reaktionsprodukt, nicht umgesetztem Edukt und dem Katalysator können im Gasabscheider 8 von leicht flüchtigen Bestandteilen durch Ausschleusung in Leitung 9 befreit werden. Für das nachfolgend aufgeführte Beispiel war diese Leitung geschlossen.

Der nach der Entgasung 8 anfallende Flüssigkeitsstrom 10 wird in einen Phasentrennbehälter 11 geleitet. Hier wird die wässrige Katalysatorphase 2 abgetrennt und erneut dem Kreislauf zugeführt. Die über ein Wehr gelaufene organische Phase, die das Reaktionsprodukt enthält, wird aus Leitung 12 entnommen.

Die Reaktionswärme kann über außerhalb des Reaktors liegende Wärmetauscher 13, 14 und 15 abgeführt werden.

Das Beispiel beschreibt die Aldolkondensation von Aceton (Ac) und Pentanal (PAL) zu 3-Octen-2-on (3-ON). Die Bildung der Nebenprodukte 4-Methyl-3-penten-2-on (4-MP), 4-Hydroxy-4-methyl-3-pentan-2-on (4-HMP), 4-Hydroxy-3-octan-2-on (4-HON), 2-Propyl-2-heptenal (2-PHL) sowie den sonstigen Hochsiedern (HS) sind in der nachfolgenden Tabelle in Massen-% angegeben.
Der Reaktor wurde mit einer Katalysatorbelastung von 400 kg/h bei einer Temperatur von 80°C bei Eigendruck der Reaktionsteilnehmer durchströmt.

**Tabelle 1:**

| | |
|---|---|
| Katalysator [g] | 4981 |
| c NaOH [Massen-%] | 4,0 |
| Wasser [Massen-%] | 91,8 |
| Aceton [Massen-%] | 4,2 |
| | |
| Edukt [l/h] | 4,28 |
| Ac [Massen-%] | 48,04 |
| PAL [Massen-%] | 51,96 |

Folgendes Ergebnis wurde erzielt:

**Tabelle 2:**

| | |
|---|---|
| RZA[t/m³/h] | 2,1 |
| U [%] | 0,95 |
| S [%] | 64,0 % |
| B | 14,72 |
| | |
| Ac [ Massen-%] | 22,52 |
| PAL [ Massen-%] | 3,04 |
| 4-MP [ Massen-%] | 0,35 |
| 4-HMP [ Massen-%] | 0,23 |
| 3-ON [ Massen-%] | 47,46 |
| 4-HON [ Massen-%] | 8,03 |
| 2-PHL [ Massen-%] | 9,49 |
| HS [ Massen-%] | 8,88 |

In der Tabelle 2 bezieht sich die Selektivität auf die Bildung von 3-Octen-2-on, bezogen auf die Summe aus 3-Octen-2-on und 4-Hydroxy-3-octan-2on beträgt die Selektivität 74 %.

### Beispiel 2: Hydrierung von 3-Octen-2-on zu 2-Octanol

Ein Liter eines Gemisches, das aus dem Reaktionsaustrag von Beispiel 1 durch Abdestillation der Leichtsieder, Aceton und Pentanal, erhalten wurde, wurde in einer Kreislaufapparatur bei 160 °C und 25 bar absolut an 100 g eines Cu/Cr/Ni-Katalysators auf Al₂O₃-Träger in der Flüssigphase 3 Stunden lang hydriert. Die Eduktanalyse und die Zusammensetzung des Hydrierungsproduktes nach 3 Stunden Versuchszeit sind in Tabelle 3 wiedergegeben.

**Tabelle 3:**

| Komponente | Edukt für Hydrierung (Massen-%) | Produkt der Hydrierung (Massen-%) |
|---|---|---|
| Aceton | 0,01 | 0,01 |
| Pentanal | 0,25 | 0,00 |
| Isopropanol | 0,00 | 0,95 |
| 1-Pentanol | 0,00 | 1,73 |
| 3-Octen-2-on | 63,65 | 0,00 |
| 2-Octanol | 0,00 | 73,25 |
| 4-Hydroxy-3-octan-2-on | 10,79 | 0,00 |
| 3-Octan-2-on | 0,00 | 1,05 |
| 4-Methyl-3-penten-2-on | 0,48 | 0,00 |
| 4-Hydroxy-4-Methyl-3-penten-2-on | 0,35 | 0,05 |
| 4-Methyl-2-pentanol | 0,00 | 0,72 |
| 2-Propyl-2-heptanal | 12,72 | 0,00 |
| 2-Propyl-heptanol | 0,00 | 12,15 |
| Hochsieder | 11,75 | 10,09 |

Wie man aus der Tabelle 3 entnehmen kann, werden 3-Octen-2-on und 4-Hydroxy-3-octen-2-on mit einer hohen Selektivität (> 97 % ) zum gewünschten Wertprodukt 2-Octanol hydriert.

### Beispiel 3: Dehydratisierung von 2-Octanol zu 1-Octen :

Der Hydrierungsaustrag aus dem Beispiel 2 wurde nach einer destillativen Entfernung der Leichtsieder (Pentanol, Isopropanol) und Hochsieder durch eine Labordestillation als Edukt mit rd. 98 Gew.-% 2-Octanol und rd. 2 Gew.-% Hochsieder für die Dehydratisierung in einem Durchfluß-Festtbettreaktor in Gegenwart eines mit Natronlauge modifizerten Zirkonoxids (ZrO₂ mit 1 Gew.-% Na₂O) eingesetzt..
Vor dem Eintritt in den Reaktor wurde das flüssige Edukt in einem vorgeschalteten Verdampfer bei 220 °C verdampft. Bei einer Reaktionstemperatur von 325 °C im Reaktor wurden stündlich 20,1 g (= 24,5 ml) Edukt durch 35,7 g (= 30 ml) Katalysator in Tablettenform in der Gasphase, entsprechend einem LHSV-Wert von 0,82 h⁻¹, durchgeleitet. Das gasförmige Produkt wurde.in einem Kühler abgekühlt und in flüssiger Form in einer Glasvorlage gesammelt.
Die GC-Analyse des Dehydratisierungsproduktes sind in Tabelle 2 wiedergegeben.

**Tabelle 4:**

| Dehydratisierung von 2-Octanol an ZrO₂-Katalysator | |
|---|---|
| Komponente | Podukte der 2-Octanol-Spaltung (Gew-%) |
| 1-Octen | 57,60 |
| tr-4-Octen | 0,00 |
| tr-3-Octen/cis-4-Octen | 0,01 |
| cis-3-Octen | 0,02 |
| tr-2-Octen | 1,73 |
| cis-2-Octen | 0,99 |
| 2-Octanon | 7,10 |
| 2-Octanol | 29,55 |
| Di-octylether | 0,25 |
| Hochsieder | 2,75 |

Wie man aus der Tabelle 4 entnehmen kann, wird das 2-Octanol mit einer hohen 1-Octen-Selektivität (> 95 % ) zum gewünschten Wertprodukt 1-Octen dehydratisiert. Nach einer destillativen Abtrennung des Wertproduktes und der Nebenprodukte kann das nicht umgesetzte 2-Octanol in den Dehydratisierungsreaktor zurückgeführt werden. Das als Nebenprodukt gebildete 2-Octanon kann zu 2-Octanol hydriert werden.

## Patentansprüche

1. Verfahren zur Herstellung von 1-Olefinen mit 7 bis 24 C-Atomen aus Aldehyden mit 4 bis 21 C-Atomen,
**dadurch gekennzeichnet,**
**dass** ein Aldehyd mit Aceton zum α,β-ungesättigten Keton kondensiert wird, wobei die Aldolisierung in einem Rohrreaktor mit einem Belastungsfaktor größer 0,8 durchgeführt wird,
**dass** das ungesättigte Keton zum gesättigten Alkohol hydriert wird und
**dass** aus dem gesättigten Alkohol Wasser abgespaltet wird.

2. Verfahren nach einem der Ansprüche 1,
**dadurch gekennzeichnet,**
**dass** die Hydrierung des α,β-ungesättigten Keton in der Flüssigphase durchgeführt wird.

3. Verfahren nach einem der Ansprüche 1 oder 2,
**dadurch gekennzeichnet,**
**dass** das α,β-ungesättigten Keton an einem im Festbett angeordneten Katalysator, der die Metalle Kupfer, Chrom und Nickel enthält, zum entsprechenden gesättigten Alkohol hydriert wird.

4. Verfahren nach einem der Ansprüche 1 bis 3,
**dadurch gekennzeichnet,**
**dass** die Bildung des 1-Olefins durch Dehydratisierung des 2-Alkohols an einem im Festbett angeordneten Katalysator in der Gas- oder Gas-Flüssig-Mischphase erfolgt.

5. Verfahren nach einem der Ansprüche 1 bis 4,
**dadurch gekennzeichnet,**
**dass** 1-Octen aus n-Pentanal hergestellt wird.

6. Verwendung der nach einem der Ansprüche 1 bis 5 gewonnenen Olefine als Comonomer.

7. Verwendung der nach einem der Ansprüche 1 bis 5 gewonnenen Olefine zur Herstellung von tertiären Carbonsäuren.

## Claims

1. A process for preparing a 1-olefin having from 7 to 24 carbon atoms from an aldehyde having from 4 to 21 carbon atoms, **characterized in that** an aldehyde is condensed with acetone to form an α,β-unsaturated ketone, the aldolization being carried out in a tube reactor having a loading factor of greater than 0.8, **in that** the unsaturated ketone is hydrogenated to form the saturated alcohol, and **in that** water is eliminated from the saturated alcohol.

2. A process according to claim 1, **characterized in that** the hydrogenation of the α,β-unsaturated ketone is carried out in the liquid phase.

3. A process according to either of claims 1 and 2, **characterized in that** the α,β-unsaturated ketone is hydrogenated over a fixed-bed catalyst comprising the metals copper, chromium and nickel to form the corresponding saturated alcohol.

4. A process according to any one of claims 1 to 3, **characterized in that** the formation of the 1-olefin by dehydration of the 2-alcohol is carried out in the gas phase or a gas/liquid mixed phase over a fixed-bed catalyst.

5. A process according to any one of claims 1 to 4, **characterized in that** 1-octene is prepared from n-pentanal.

6. The use of an olefin obtained according to any one of claims 1 to 5 as comonomer.

7. The use of an olefin obtained according to any one of claims 1 to 5 for preparing teriary carboxylic acids.

## Revendications

1. Procédé pour la préparation de 1-oléfines comprenant 7 à 24 atomes de carbone à partir d'aldéhydes comprenant 4 à 21 atomes de carbone, **caractérisé en ce qu'**on condense un aldéhyde avec de l'acétone en cétone α,β-insaturée, l'aldolisation étant réalisée dans un réacteur tubulaire avec un facteur de charge supérieur à 0,8, **en ce que** la cétone insaturée est hydrogénée en alcool saturé et **en ce qu'**on dissocie de l'eau de l'alcool saturé.

2. Procédé selon la revendication 1, **caractérisé en ce que** l'hydrogénation de la cétone α,β-insaturée est réalisée dans la phase liquide.

3. Procédé selon l'une quelconque des revendications 1 ou 2, **caractérisé en ce que** la cétone α,β-insaturée est hydrogénée en alcool saturé correspondant sur un catalyseur disposé dans un lit fixe, qui contient les métaux cuivre, chrome et nickel.

4. Procédé selon l'une quelconque des revendications 1 à 3, **caractérisé en ce que** la formation de la 1-oléfine est réalisée par déshydratation du 2-alcool en phase gazeuse ou en phase mixte gazeuse-liquide sur un catalyseur disposé dans un lit fixe.

5. Procédé selon l'une quelconque des revendications 1 à 4, **caractérisé en ce qu'**on prépare du 1-octène à partir de n-pentanal.

6. Utilisation de l'oléfine obtenue selon l'une quelconque des revendications 1 à 5 comme comonomère.

7. Utilisation de l'oléfine obtenue selon l'une quelconque des revendications 1 à 5 pour la préparation d'acides carboxyliques tertiaires.
